(19) Europäisches Patentamt
European Patent Office
Office européen des brevets

(11) Numéro de publication : **0 347 328 B1**

(12) # FASCICULE DE BREVET EUROPEEN

(45) Date de publication du fascicule du brevet : **20.03.91 Bulletin 91/12**

(51) Int. Cl.⁵ : **A61K 7/06**, A61K 7/08

(21) Numéro de dépôt : **89401695.5**

(22) Date de dépôt : **16.06.89**

(54) **Composition pour induire et stimuler la croissance des cheveux et/ou freiner leur chute, à base d'un agent tensio-actif amphotère et d'un dérivé pyrimidine.**

(30) Priorité : **17.06.88 LU 87249**

(43) Date de publication de la demande : **20.12.89 Bulletin 89/51**

(45) Mention de la délivrance du brevet : **20.03.91 Bulletin 91/12**

(84) Etats contractants désignés : **BE CH DE FR GB IT LI NL SE**

(56) Documents cités :
**WO-A-88/01863**
**DE-A- 3 615 396**

(56) Documents cités :
**SOAP, PERFUMERY & COSMETICS, vol. 59, no. 11, novembre 1986, pages 617,619, Londres, GB; P. ALEXANDER: "The miranol amphoteric surfactants: chemistry & applications part 2"**

(73) Titulaire : **L'OREAL**
**14, Rue Royale**
**F-75008 Paris (FR)**

(72) Inventeur : **Grollier, Jean-François**
**16bis, Boulevard Morland**
**F-75004 Paris (FR)**

(74) Mandataire : **Casalonga, Axel et al**
**BUREAU D.A. CASALONGA - JOSSE**
**Morassistrasse 8**
**W-8000 München 5 (DE)**

**EP 0 347 328 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une composition pour induire et stimuler la croissance des cheveux et/ou freiner leur chute contenant, en association, au moins un dérivé de pyrimidine et au moins un agent tensio-actif amphotère.

On recherche depuis de nombreuses années dans l'industrie cosmétique ou pharmaceutique des compositions permettant de supprimer ou de réduire l'alopécie et notamment d'induire ou de stimuler la croissance des cheveux et/ou de diminuer leur chute.

On a déjà proposé, dans cette optique, des composés tels que l'amino-6 dihydro-1,2 hydroxy-1 imino-2 pipéridino-4 pyrimidine ou Minoxidil et ses dérivés. De tels composés sont décrits, notamment, dans le brevet US-A-4 139 619.

Ces composés et plus particulièrement le Minoxidil, ont été préconisés en application topique sur le cuir chevelu sous forme de lotion, dont l'application n'est pas suivie d'un rinçage.

Par ailleurs et compte tenu des difficultés pour solubiliser ces composés, on a utilisé jusqu'à présent des solvants dont les effets sur les cheveux des personnes traitées sont le plus souvent peu cosmétiques, d'autant plus que le traitement préconisé nécessite plusieurs applications de la lotion à une fréquence journalière.

On appelle "peu cosmétique" un traitement pouvant coller, poisser ou graisser les cheveux.

Il est apparu souhaitable et avantageux de rechercher l'application du Minoxidil grâce à un milieu solubilisant tensio-actif, c'est-à-dire un milieu s'éliminant facilement à l'eau, tel que par exemple une solution moussante ou un shampooing, qu'on applique sur le cuir chevelu et les cheveux mouillés, qu'on laisse éventuellement poser quelques minutes après massage et qu'on élimine par rinçage à l'eau.

Cette utilisation serait d'autant plus intéressante que les cheveux des sujets à tendance alopécique sont souvent gras et qu'il est souhaitable d'utiliser une composition qui lave, c'est-à-dire qui débarrasse le cuir chevelu et les cheveux des sécrétions de sébum ou autres impuretés qui poissent les cheveux et peuvent nuire à la pénétration de l'agent actif, tout en déposant dans le même temps la quantité d'agents actifs nécessaire pour agir au niveau du freinage de la chute des cheveux et/ou de l'induction et de la stimulation de leur repousse.

On a pensé utiliser à cette fin des agents tensio-actifs, peu agressifs, vis-à-vis du cuir chevelu, notamment lorsqu'on laisse poser quelque temps la composition avant de la rincer. Parmi ces agents tensio-actifs, on peut citer les agents tensio-actifs amphotères.

La demanderesse a cependant constaté que la stabilité dans le temps des dérivés de pyrimidine et en particulier du Minoxidil dans de tels milieux, n'était pas satisfaisante.

Elle a découvert, de façon surprenante, que le problème de la stabilité dans le temps pouvait être résolu grâce à l'utilisation d'une famille particulière d'agents tensio-actifs amphotères constitués par les amphocarboxyglycinates ou amphocarboxypropionates.

Les compositions conformes à l'invention présentent donc l'avantage d'être particulièrement stables dans le temps sans essentiellement de perte de principe actif.

La présente invention a donc pour objet une composition destinée à induire ou stimuler la croissance des cheveux et/ou freiner leur chute, à base d'un agent tensio-actif amphotère de la famille des amphocarboxyglycinates ou des amphocarboxypropionates et de dérivés de pyrimidine.

Un autre objet de l'invention est constitué par le procédé de traitement des cheveux et du cuir chevelu grâce à une telle composition.

D'autres objets de l'invention apparaîtront à la lecture de la description et des exemples qui suivent.

La composition, objet de l'invention, destinée à induire et stimuler la croissance des cheveux et freiner leur chute, est essentiellement caractérisée par le fait qu'elle contient dans un milieu physiologiquement acceptable, au moins un agent tensio-actif amphotère choisi parmi les amphocarboxyglycinates répondant à la formule :

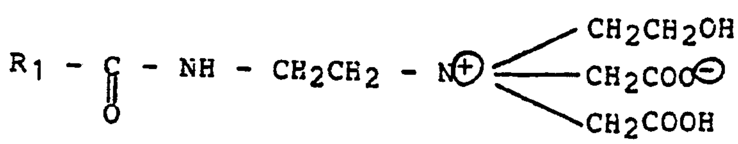

(I)

dans laquelle $R_1$ désigne un radical alkyle dérivé du coprah, un radical heptyle, nonyle ou undécyle ; ou les amphocarboxypropionates de formule :

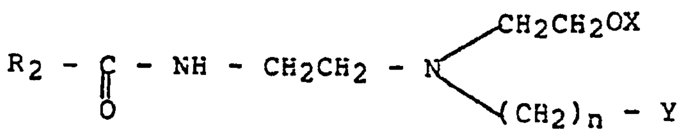

(II)

dans laquelle n = 1 ou 2 ;

X désigne un radical $CH_2CH_2COOH$, hydrogène ;

Y désigne COOH ou

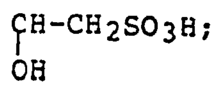

$R_2$ désigne un radical alkyle dérivé du coprah, un radical alkyle saturé en $C_7$, $C_9$, $C_{11}$, $C_{13}$, $C_{17}$ et la forme iso, un radical en $C_{17}$ insaturé ou encore un radical alkyle dérivé de l'huile de lin ;

ainsi que leurs sels physiologiquement acceptables ;

et au moins un composé répondant à la formule :

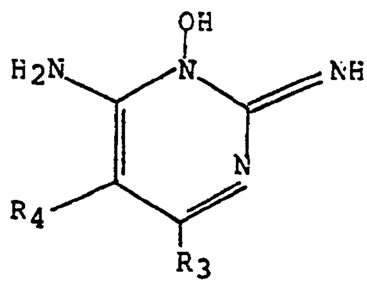

(III)

dans laquelle $R_3$ désigne un groupement

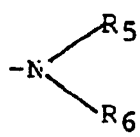

dans lequel :

$R_5$ et $R_6$ sont choisi parmi :

l'hydrogène, un groupement alkyle ayant de préférence 1 à 4 atomes de carbone, alcényle, alkylaryle ou cycloalkyle inférieur ;

$R_5$ et $R_6$ peuvent également former un hétérocycle avec l'atome d'azote auquel ils sont liés, cet hétérocycle étant choisi parmi les groupements aziridinyle, azétidinyle, pyrrolidinyle, pipéridinyle, hexahydroazépinyle, heptaméthylèneimine, octaméthylèneimine, morpholine et alkyle inférieur-4-pipérazidinyle ;

les groupements hétérocycliques pouvant être substitués sur les atomes de carbone par un à trois groupements alkyle inférieur, hydroxy ou alcoxy ;

le groupement $R_4$ est choisi parmi l'hydrogène, un groupement alkyle, alcényle, alcoxyalkyle, cycloalkyle, aryle, alkylaryle, arylalkyle, alkylarylalkyle, alcoxyarylalkyle et haloarylalkyle inférieur, ainsi que les sels d'addition d'acides cosmétiquement ou pharmaceutiquement acceptables.

Dans la formule (III), les groupements alkyle ou alcoxy désignent de préférence un groupement ayant 1 à 4 atomes de carbone ; le groupement alcényle désigne de préférence un groupement ayant 2 à 5 atomes de carbone ; le groupement aryle désigne de préférence phényle ; le groupement cycloalkyle désigne de préférence un groupement ayant 4 à 6 atomes de carbone.

Les composés préférés de formule (III) sont plus particulièrement choisis parmi les composés dans lesquels $R_4$ désigne hydrogène et $R_3$ désigne un groupement :

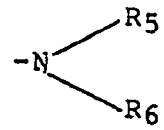

dans lequel $R_5$ et $R_6$ forment un cycle pipéridinyle ou bien des groupements éthyle ainsi que leurs sels, tels que par exemple le sulfate.

Parmi ces composés, l'amino-6 dihydro-1,2 hydroxy-1 imino-2 pipéridino-4 pyrimidine, encore appelé Minoxidil, est particulièrement préféré.

Parmi les composés particulièrement préférés, répondant à la formule (I), définis ci-dessus, on utilise plus particulièrement les composés de formule (I) dans lesquels $R_1$ désigne un radical alkyle dérivé du coprah, et en particulier le composé vendu sous la dénomination MIRANOL C2M conc, et mentionné dans le dictionnaire CTFA, 3ème édition, 1982, sous la dénomination "COCOAMPHOCARBOXY-GLYCINATE".

Les composés de formule (II) préférés sont plus particulièrement choisis parmi les composés dans lesquels :

$R_2$ désigne un radical alkyle dérivé du coprah
n = 2
X = $CH_2CH_2COONa$
Y = COONa

et plus particulièrement le composé répondant à la formule (II), vendu sous la dénomination MIRANOL C2M SF par la Société MIRANOL et désigné dans le dictionnaire CTFA, 3ème édition, 1982, sous la dénomination COCOAMPHOCARBOXYPROPIONATE.

Les sels des composés de formules (I) ou (II) sont en particulier des sels de métaux alcalins tels que de sodium ou alcalino-terreux.

Une composition particulièrement préférée, conforme à l'invention, est constituée par une composition contenant dans un milieu physiologiquement acceptable, au moins l'amino-6 dihydro-1,2 hydroxy-1 imino-2 pipéridino-4 pyrimidine et une agent tensio-actif amphotère de formule (I), dans laquelle $R_1$ désigne un radical alkyle dérivé du coprah.

Les compositions conformes à l'invention sont des compositions lavantes et moussantes, présentant, outre une excellente stabilité dans le temps, aucune irritation du cuir chevelu, et laissent les cheveux dans un état cosmétique parfaitement acceptable.

Les compositions conformes à l'invention contiennent le dérivé de pyrimidine de formule (III), dans des proportions comprises de préférence entre 0,05 et 6% en poids par rapport au poids total de la composition, et de préférence entre 0,1 et 3% en poids, et plus particulièrement entre 0,3 et 2% en poids.

L'agent tensio-actif amphotère de formules (I) ou (II) est utilisé de préférence dans des proportions comprises entre 1 et 30% en poids par rapport au poids total de la composition, et de préférence entre 3 et 20%.

Le milieu physiologiquement acceptable peut être un milieu acceptable sur le plan cosmétique ou pharmaceutique et être constitué par un milieu aqueux ou un mélange d'eau et d'un solvant cosmétiquement ou pharmaceutiquement acceptable, éventuellement épaissi.

A titre de solvants, on peut utiliser notamment des alcools inférieurs en $C_1$-$C_4$, tels que l'alcool éthylique, l'alcool isopropylique et l'alcool tertiobutylique ; des alkylèneglycols comme le propylène glycol et des alkyléthers de mono- et de dialkylène glycol, tels que le monoéthyléther d'éthylèneglycol, le monométhyléther de propylèneglycol et le monoéthyléther de diéthylèneglycol.

Les solvants sont de préférence utilisés dans des proportions comprises entre 0,5 et 10% en poids par rapport au poids total de la composition.

Ces compositions peuvent être épaissies avec des agents épaississants bien connus dans l'état de la technique et plus particulièrement les hétérobiopolysaccharides tels que la gomme de xanthane ou les scléroglucanes, les dérivés de la cellulose, les polymères acryliques réticulés ou non.

Ces agents épaississants sont utilisés de préférence dans des proportions comprises entre 0,1 et 5% en poids par rapport au poids total de la composition.

Les compositions conformes à l'invention peuvent également contenir des agents tensio-actifs non ioniques et plus particulièrement des agents choisis parmi les polyhydroxypropyléthers, les éthers d'alcool gras polyoxyéthylénés, les alkylphénols oxyéthylénés, ou des mélanges d'alcools gras et d'alcools gras polyoxyéthylénés, dans des proportions pouvant aller de préférence jusqu'à 10% en poids par rapport au poids total de la composition.

Les agents tensio-actifs non ioniques de la famille des poly(hydroxypropyléther) sont plus particulièrement choisis parmi :

(1) les composés de formule :

$$R_7O - (CH_2 - \underset{\displaystyle CH_2OH}{\underset{|}{C}}HO)_m\!\!-\!\!-\!\!-H \qquad (IV)$$

dans laquelle $R_7$ désigne un radical ou un mélange de radicaux alkyle contenant 10 à 14 atomes de carbone et m est un nombre entier ou décimal de 2 à 10, de préférence de 3 à 6 ;

(2) les composés de formule :

$$R_8\text{-}CONH\text{-}CH_2\text{-}CH_2\text{-}O\text{-}CH_2\text{-}CH_2\text{-}O(CH_2\text{-}CHOH\text{-}CH_2\text{-}CH_2\text{-}O)_n \quad H \qquad (V)$$

dans laquelle $R_8$ désigne un radical ou un mélange de radicaux alkyle et/ou alcényle, ayant de 11 à 17 atomes de carbone et n désigne un nombre entier ou décimal de 1 à 5 et de préférence de 1,5 à 4 ;

(3) les composés de formule :

$$R_9\text{-}CHOH\text{-}CH_2\text{-}O(CH_2\text{-}CHOH\text{-}CH_2\text{-}O)_p\!\!-\!\!-\!\!-H \qquad (VI)$$

dans laquelle $R_9$ désigne un radical aliphatique, cycloaliphatique, arylaliphatique, ayant de préférence 7 à 21 atomes de carbone, et leurs mélanges, les chaînes aliphatiques désignant en particulier des chaînes alkyle pouvant comporter 1 à 6 groupements éther, thioéther et/ou hydroxyméthylène, et p est un nombre compris entre 1 et 10 inclus ;

(4) les composés préparés par condensation en catalyse acide, de 2 à 10 et de préférence de 2,5 à 6 moles de glycidol par mole d'alcool ou d'alpha-diol contenant 10 à 14 atomes de carbone ;

(5) les composés préparés par (poly)addition de monochlorhydrine de glycérol sur un composé organique poly(hydroxylé) en présence d'une base forte et élimination d'eau.

Des agents tensio-actifs non ioniques plus particulièrement préférés, conformément à l'invention, sont choisis parmi :

(i) les composés polyhydroxypropyléther de formules :

$$C_{12}H_{25}O - (CH_2 - \underset{\displaystyle CH_2OH}{\underset{|}{CH}} - O)_{4,2}\!\!-\!\!-\!\!-H \qquad (VII)$$

$$R_7O - (CH_2 - \underset{\displaystyle CH_2OH}{\underset{|}{CH}} - O)_{3,75}\!\!-\!\!-\!\!-H \qquad (VIII)$$

dans laquelle $R_7$ désigne un mélange de radicaux alkyle $C_{10}H_{21}$ et $C_{12}H_{25}$.

(ii) $$R_8\text{-}CONH\text{-}CH_2\text{-}CH_2\text{-}O\text{-}CH_2\text{-}CH_2\text{-}O\text{-}(CH_2\text{-}CHOH\text{-}CH_2\text{-}O)_{3,5}\!\!-\!\!-\!\!-H \qquad (IX)$$

dans laquelle $R_8$ désigne un mélange de radicaux comprenant les radicaux alkyle et alcényle suivants : $C_{11}H_{23}$, $C_{13}H_{27}$, ou des radicaux dérivés des acides gras du coprah, ou radical dérivé de l'acide oléïque ;

(iii) l'agent tensio-actif non ionique du type poly hydroxypropyléther préparé par condensation en catalyse alcaline de 3,5 moles de glycidol sur un mélange d'alpha-diols ayant de 11 à 14 atomes de carbone ;

(iv) le composé du type polyhydroxypropyléther obtenu par condensation de la monochlorhydrine (2,5 moles) en présence de soude sur le docécanediol-1,2.

Les agents tensio-actifs non ioniques de la famille des polyéthylèneglycoléthers sont plus particulièrement

choisis parmi les produits de condensation d'un mélange d'alcools gras ayant 10 à 22 atomes de carbone avec 1 à 24 moles d'oxyde d'éthylène.

Les alkylphénols oxyéthylénés sont plus particulièrement choisis parmi les alkyle en $C_8$-$C_9$ phénol oxyéthylénés avec 1 à 100 moles d'oxyde d'éthylène.

Les mélanges d'alcools gras et d'alcools gras polyoxyéthylénés sont choisis en particulier parmi les mélanges d'alcools gras en $C_{10}$-$C_{22}$ et d'alcools en $C_{10}$-$C_{22}$ oxyéthylénés avec 2 à 50 moles d'oxyde d'éthylène, dans les proportions relatives de 10 : 90 à 90 : 10. A titre d'exemple, on peut citer plus particulièrement les mélanges d'alcool cétylstéarylique à 80% et d'alcool cétylstéarylique oxyéthyléné à 33 moles d'oxyde d'éthylène (20%).

Les compositions conformes à l'invention peuvent éventuellement renfermer des ingrédients classiquement utilisés dans les compositions cosmétiques ou pharmaceutiques, destinés à une application topique et plus particulièrement des agents conservateurs, des agents complexants, des colorants, des agents alcalinisants ou acidifiants, des parfums, des polymères.

Le pH de ces compositions est généralement compris entre 4 et 9 et de préférence entre 7 et 8,5.

Elles peuvent se présenter sous forme de lotions épaissies ou non, de gels, de crèmes, d'émulsions et être pressurisées dans des dispositifs aérosols.

Les compositions conformes à l'invention sont plus particulièrement destinées à être utilisées pour le lavage des cheveux sous forme de shampooing.

Le procédé de traitement pour lutter contre la chute des cheveux et/ou stimuler leur croissance consiste à appliquer la composition sur les cheveux mouillés, à procéder à un massage, à éventuellement la laisser en contact quelques minutes avec les cheveux, à rincer à l'eau et à appliquer éventuellement une nouvelle fois le shampooing suivi d'un nouveau rinçage à l'eau.

Le procédé conforme à l'invention présente les caractéristiques d'un traitement thérapeutique des alopécies dans la mesure où la composition agit sur les mécanismes biologiques et notamment sur le cycle pilaire en corrigeant le dysfonctionnement de celui-ci.

Il présente également les caractéristiques d'un traitement cosmétique mais il permet en même temps d'embellir les cheveux et le cuir chevelu.

Les exemples suivants sont destinés à illustrer l'invention.

EXEMPLE 1

On prépare un shampooing non irritant, antichute, de composition suivante :

| | | |
|---|---|---|
| - Minoxidil | | 0,25 g |
| - Produit dénommé "COCOAMPHOCARBOXYGLYCINATE" (CTFA, 3ème édition, 1982), vendu sous la dénomination MIRANOL C2M conc, par la Société MIRANOL en solution aqueuse à 38% de matière active | | 15,0 g MA |
| - Parfum, séquestrant | qs | |
| - Eau | qsp | 100,0 g |

Ce shampooing présente une excellente stabilité dans le temps sans perte substantielle de principe actif (Minoxidil).

EXEMPLE 2

On prépare un shampooing non irritant, antichute, de composition suivante :

| | | | |
|---|---|---|---|
| - Minoxidil | | 0,25 | g |
| - Produit dénommé "COCOAMPHOCARBOXY-GLYCINATE" (CTFA, 3ème édition, 1982), vendu sous la dénomination MIRANOL C2M conc, par la Société MIRANOL en solution aqueuse à 38% de matière active | | 6,0 | g MA |
| - Parfum, séquestrant | qs | | |
| - Eau | qsp | 100,0 | g |

Ce shampooing peu moussant présente une excellente stabilité dans le temps sans perte substantielle de principe actif (Minoxidil).

EXEMPLE 3

On prépare un shampooing pour le traitement de la chute des cheveux de composition suivante :

| | | | |
|---|---|---|---|
| - Minoxidil | | 0,25 | g |
| - Composé de formule (II) dans laquelle :<br>$R_2$ = undécyle, n = 2<br>X = $CH_2CH_2$-COONa<br>Y = -COONa<br>dénommé "Lauroamphocarboxypropionate" (CTFA, 3ème édition, 1982), vendu sous la dénomination MIRANOL H2M/SF Conc. par la Société MIRANOL à la concentration de 39% de matière active (MA) | | 15,0 | g MA |
| - Séquestrant | | 0,2 | g |
| - Eau | qsp | 100,0 | g |

Ce shampooing présente une excellente stabilité dans le temps sans perte de principe actif.

EXEMPLE 4

On prépare un shampooing pour le traitement de la chute des cheveux de composition suivante :

| | | | |
|---|---|---|---|
| - Minoxidil | | 0,26 | g |
| - Composé de formule (II) dans laquelle :<br>$R_2$ = alkyle dérivé du coprah<br>n = 2<br>X = $-CH_2CH_2-COONa$<br>Y = $-COONa$<br>dénommé "Cocoamphocarboxypropionate" CTFA, 3ème édition, 1982), vendu sous la dénomination MIRANOL C2M/SF Conc. par la Société MIRANOL à la concentration de 39% de matière active (MA) | | 15,0 | g MA |
| - Séquestrant | | 0,2 | g |
| - Eau | qsp | 100,0 | g |

Ce shampooing présente une excellente stabilité dans le temps sans perte de principe actif.

EXEMPLE 5

On prépare un shampooing pour le traitement de la chute des cheveux de composition suivante :

| | | | |
|---|---|---|---|
| - Minoxidil | | 0,25 | g |
| - Composé de formule (II) dans laquelle :<br>$R_2$ = undécyle<br>n = 1<br>X = H<br>Y = $-\underset{OH}{\underset{\vert}{CH}}-CH_2-SO_3Na$<br>dénommé "Lauroamphopropylsulfonate" CTFA, 3ème édition, 1982), vendu sous la dénomination MIRANOL HS Conc. par la Société MIRANOL à la concentration de 38% de matière active (MA) | | 15,0 | g MA |
| - Séquestrant | | 0,3 | g |
| - Eau | qsp | 100,0 | g |

Ce shampooing présente une excellente stabilité dans le temps sans perte de principe actif.

EXEMPLE 6

On prépare un shampooing pour le traitement de la chute des cheveux de composition suivante :

| | | | |
|---|---|---|---|
| - Minoxidil | | 0,27 | g |
| - Sel sodique du composé de formule (I) dans laquelle $R_1$ = undécyle dénommé "Lauroamphocarboxyglycinate" (CTFA, 3ème édition, 1982), vendu sous la dénomination MIRANOL H2M Conc. par la Société MIRANOL à la concentration de 38% de matière active (MA) | | 15,0 | g MA |
| - Séquestrant | | 0,2 | g |
| - Eau | qsp | 100,0 | g |

Ce shampooing présente une excellente stabilité dans le temps sans perte de principe actif.

EXEMPLE 7

On prépare un shampooing pour le traitement de la chute des cheveux de composition suivante :

| | | | |
|---|---|---|---|
| - Minoxidil | | 0,27 | g |
| - Composé de formule (II) dans laquelle :<br>$R_2$ = undécyle<br>n = 1<br>X = H<br>Y = -COONa<br>dénommé "Lauroamphoglycinate" (CTFA, 3ème édition, 1982), vendu sous la dénomination MIRANOL HM Conc. par la Société MIRANOL à la concentration de 35% de matière active (MA) | | 15,0 | g MA |
| - Séquestrant | | 0,2 | g |
| - Eau | qsp | 100,0 | g |

Ce shampooing présente une excellente stabilité dans le temps sans perte de principe actif.

EXEMPLE 8

On prépare un shampooing pour le traitement de la chute des cheveux de composition suivante :

| | | |
|---|---|---|
| - Minoxidil | | 0,25 g |
| - Produit dénommé "Cocoamphocarboxy glycinate" (CTFA, 3ème édition, 1982) vendu sous la dénomination MIRANOL C2M Conc. par la Société MIRANOL en solution aqueuse à 38% de matière active (MA) | | 3,8 g MA |
| - Tensio-actif non ionique poly (hydroxypropyléther) obtenu par condensation de la monochlorhydrine du glycérol (2,5 moles) en présence de soude sur le dodécanediol-1,2 selon la brevet français FR-25 74 786 | | 12,6 g |
| - Séquestrant | | 0,2 g |
| - Eau | qsp | 100,0 g |

Ce shampooing est particulièrement non irritant et présente une excellente stabilité dans le temps sans perte de principe actif.

## Revendications

1. Composition lavante et moussante pour induire et stimuler la croissance des cheveux et/ou freiner leur chute, caractérisée par le fait qu'elle contient dans un milieu physiologiquenent acceptable, au moins un agent tensio-actif amphotère choisi parmi les amphocarboxyglycinates de formule :

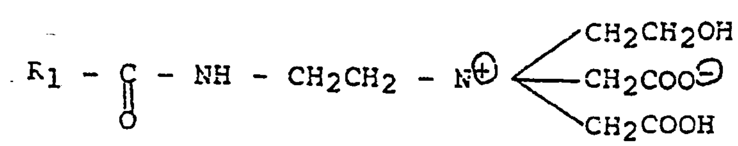

(I)

dans laquelle $R_1$ peut désigner un radical alkyle dérivé du coprah, un radical heptyle, nonyle ou indécyle ; et les amphocarboxypropionates répondant à la formule :

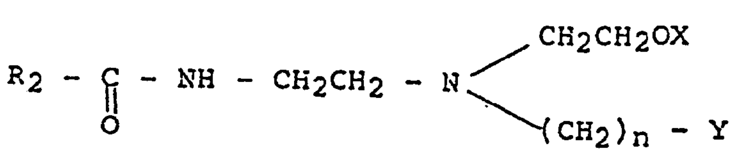

(II)

dans laquelle :

n = 1 ou 2 ;

X désigne $-CH_2CH_2COOH$, ou hydrogène ;

Y désigne –COOH ou le radical

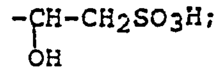

$R_2$ désigne un radical alkyle dérivé du coprah, un radical alkyle saturé en $C_7$, $C_9$, $C_{11}$, $C_{13}$, un radical alkyle en $C_{17}$ et sa forme iso, un radical en $C_{17}$ insaturé, un radical alkyle dérivé de l'huile de lin ; ainsi que les sels physiologiquement acceptables de ces composés ; et un dérivé de pyrimidine répondant à la formule :

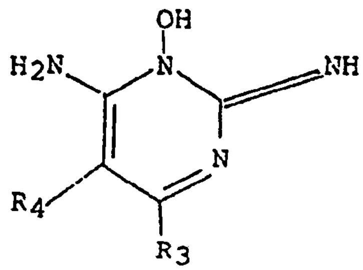

(III)

dans laquelle $R_3$ désigne un groupement

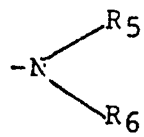

dans lequel :

$R_5$ et $R_6$ sont choisis parmi l'hydrogène, un groupement alkyle ayant de préférence 1 à 4 atomes de carbone, alcényle, alkylaryle ou cycloalkyle inférieur ;

$R_5$ et $R_6$ peuvent également former un hétérocycle avec l'atome d'azote auquel ils sont liés, cet hétérocycle étant choisi parmi les groupements aziridinyle, azétidinyle, pyrrolidinyle, pipéridinyle, hexahydroazépinyle, heptaméthylèneimine, octaméthylèneimine, morpholine et alkyle inférieur-4-pipérazidinyle, les groupements hétérocycligues pouvant être substitués sur les atomes de carbone par un à trois groupements alkyle inférieur, hydroxy ou alcoxy ;

le groupement $R_4$ est choisi parmi l'hydrogène, un groupement alkyle, alcényle, alcoxyalkyle, cycloalkyle, aryle, alkylaryle, arylalkyle, alkylarylalkyle, alcoxyarylalkyle et haloarylalkyle inférieur, ainsi, que les sels d'addition d'acides physiologiquement acceptables.

2. Composition selon la revendication 1, caractérisée par le fait que les dérivés de pyrimidine sont choisis parmi les composés de formule (III), dans laquelle $R_4$ désigne hydrogène et $R_3$ désigne un groupement :

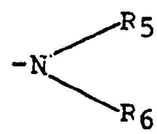

dans lequel $R_5$ et $R_6$ forment un cycle pipéridinyle ou bien un groupement éthyle ainsi que leurs sels physiologiquement acceptables.

3. Composition selon la revendication 1 ou 2, caractérisée par le fait que l'amphocarboxyglycinate de formule (I) est choisi parmi les composés de formule (I) dans lesquels $R_1$ désigne un radical alkyle dérivé du coprah.

4. Composition selon l'une quelconque des revendications 1 ou 2, caractérisée par le fait, que l'amphocarboxypropionate de formule (II) est choisi parmi les composés dans lequels :

$R_2$ désigne en radical alkyle dérivé du coprah

$n = 2$

$X = CH_2CH_2COONa$

$Y = COONa$

5. Composition selon l'une quelconque des revendications 1, 2 et 3, caractérisée par le fait qu'elle contient dans un milieu physiologiquement acceptable au moins l'amino-6 dihydro-1,2 hydroxy-1 imino-2 pipéridino-4

pyrimidine et un agent tensio-actif amphotère de formule (I), dans laquelle $R_1$ désigne un radical alkyle dérivé du coprah.

6. Composition selon l'une quelconque des revendications 1 à 5, caractérisée par le fait que les dérivés de pyrimidine de formule (III) sont présents dans les compositions conformes à l'invention dans des proportions comprises entre 0,05 et 6% en poids par rapport au poids total de la composition, et de préférence de 0,1 à 3% en poids.

7. Composition selon l'une quelconque des revendications 1 à 6, caractérisée par le fait que l'agent tensio-actif amphotère de formule (I) ou (II) est présent dans la composition dans des proportions comprises entre 1 et 30% en poids par rapport au poids total de la composition et de préférence entre 3 et 20% en poids.

8. Composition selon l'une quelconque des revendications 1 à 7, caractérisée par le fait que le milieu physiologiquement acceptable est un milieu aqueux ou un mélange d'eau et d'un solvant, cosmétiquement ou pharmaceutiquement acceptable, épaissi ou non.

9. Composition selon l'une quelconque des revendications 1 à 8, caractérisée par le fait que les solvants sont utilisés dans des proportions comprises entre 0,5 et 10% en poids par rapport au poids total de la composition.

10. Composition selon l'une quelconque des revendications 1 à 9, caractérisée par le fait, qu'elle contient des épaississants choisis parmi les hétérobiopolysaccharides, les dérivés de cellulose, les polymères acryliques réticulés ou non présents dans des proportions comprises entre 0,1 et 5% en poids par rapport au poids total de la composition.

11. Composition selon l'une quelconque des revendications 1 à 10, caractérisée par le fait que la composition contient en plus au moins un agent tensio-actif non ionique.

12. Composition selon la revendication 11, caractérisée par le fait que l'agent tensio-actif non ionique est choisi parmi les polhydroxypropyléthers, les éthers d'alcools gras polyoxyéthylénés, les alkylphénols oxyéthylénés, des mélanges d'alcools gras et d'alcools gras polyoxyéthylénés.

13. Composition selon la revendication 12, caractérisée par le fait que l'agent tensio-actif non ionique du type polyhydroxypropyléther est choisi parmi :

(i) les composés de formule :

$$R_7O-(CH_2-\underset{\displaystyle CH_2OH}{\underset{|}{CH}}O)_{\overline{m}}H \qquad (IV)$$

dans laquelle $R_7$ désigne un radical ou un mélange de radicaux alkyle contenant 10 à 14 atomes de carbone et m est un nombre entier ou décimal de 2 à 10 ;

(ii) les composés de formule :

$$R_8-CONH-CH_2-CH_2-O-CH_2-CH_2-O(CH_2-CHOH-CH_2-O)_{\overline{n}}H \qquad (V)$$

dans laquelle $R_8$ désigne un radical ou un mélange de radicaux alkyle et/ou alcényle, ayant de 11 à 17 atomes de carbone et n désigne un nombre entier ou décimal de 1 à 5 ;

(iii) les composés de formule :

$$R_9-CHOH-CH_2-O(CH_2-CHOH-CH_2-O)_{\overline{p}}H \qquad (VI)$$

dans laquelle $R_9$ désigne un radical aliphatique, cycloaliphatique, arylaliphatique, ayant de préférence 7 à 21 atomes de carbone et leurs mélanges ;

(iv) les composés préparés par condensation en catalyse acide, de 2 à 10 et de préférence de 2,5 à 6 moles de glycidol par mole d'alcool ou d'alpha-diol, contenant 10 à 14 atomes de carbone ;

(v) les composés préparés par (poly)addition de monochlorhydrine de glycérol sur un composé organique poly(hydroxylé) en présence d'une base forte et élimination d'eau.

14. Composition selon la revendication 12, caractérisée par le fait que l'agent tensio-actif non ionique de la famille des polyéthylèneglycoléthers est choisi parmi les produits de condensation d'alcools gras ayant 10 à 22 atomes de carbone avec 1 à 24 moles d'oxyde d'éthylène.

15. Composition selon la revendication 12, caractérisée par le fait que l'alkylphénol oxyéthyléné est un alkyle en $C_8$-$C_9$ phénol oxyéthyléné avec 1 à 100 moles d'oxyde d'éthylène.

16. Composition selon la revendication 12, caractérisée par le fait que le mélange d'alcools gras et d'alcools

gras polyoxyéthylénés est un mélange d'alcools gras en $C_{10}$-$C_{22}$ et d'alcools gras en $C_{10}$-$C_{22}$ oxyéthylénés avec 2 à 50 moles d'oxyde d'éthylène.

17. Composition selon l'une quelconque des revendications 1 à 16, caractérisée par le fait qu'elle contient également des agents conservateurs, des agents complexants, des colorants, des agents alcalinisants ou acidifiants, des parfums, des polymères.

18. Composition selon l'une quelconque des revendications 1 à 17, caractérisée par le fait que le pH est compris entre 4 et 9.

19. Procédé de traitement cosmétique des cheveux, caractérisé par le fait que l'on applique la composition définie dans l'une quelconque des revendications 1 à 17 sur des cheveux mouillés, que l'on procède à un massage, qu'on laisse en contact quelques minutes, qu'on rince à l'eau et qu'on applique éventuellement une nouvelle fois cette composition suivie d'un nouveau rinçage.

20. Composition selon l'une quelconque des revendications 1 à 18 pour son application dans le traitement thérapeutique de l'alopécie.

21. Utilisation de la composition selon l'une quelconque des revendications 1 à 18 pour la préparation d'un médicament destiné au traitement thérapeutique de l'alopécie.

## Ansprüche

1. Reinigende und schäumende Zusammensetzung zur Induzierung und Stimulierung des Haarwuchses und/oder zur Verringerung des Haarausfalles, dadurch gekennzeichnet, daß sie in einem physiologisch annehmbaren Milieu mindestens ein amphoteres oberflächenaktives Mittel enthält, ausgewählt aus den Amphocarboxyglycinaten der Formel :

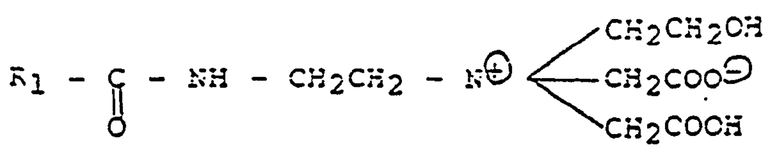

(I)

worin $R_1$ ein von Kopra abgeleitetes Radikal, ein Heptyl-, Nonyl- oder Undecylradikal bedeuten kann ; und den Amphocarboxypropionaten der Formel :

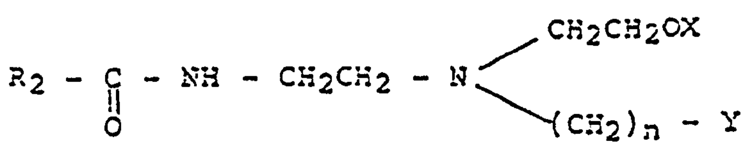

(II)

worin bedeutet :

n = 1 oder 2 ;

X $-CH_2CH_2COOH$, oder Wasserstoff ;

Y COOH oder das Radikal $-CH(OH)-CH_2SO_3H$ ;

$R_2$ ein von Kopra abgeleitetes Radikal bedeutet, ein gesättigtes $C_7$, $C_9$, $C_{11}$, $C_{13}$ -Alkylradikal, ein Alkylradikal mit $C_{17}$ und seine Isoform, ein ungesättigtes $C_{17}$ -Radikal, ein von Leinöl abgeleitetes Alkylradikal ; sowie die physiologisch annehmbaren Salze dieser Verbindungen ; und ein Pyrimidinderivat der Formel :

2

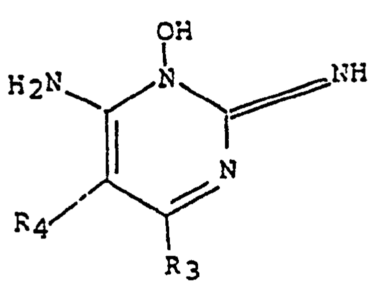

(III)

worin $R_3$ eine Gruppe

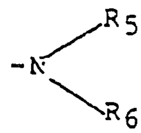

bedeutet, worin bedeuten :

$R_5$ und $R_6$ ausgewählt sind aus Wasserstoff, einer Alkylgruppe mit vorzugsweise 1 bis 4 Kohlenstoffatomen, Alkenyl, Alkylaryl, oder niedrigem Cykloalkyl ;

$R_5$ und $R_6$ zusammen mit dem Stickstoffatom, an das sie gebunden sind auch einen Heterocyklus binden können, wobei dieser Heterocyklus ausgewählt ist unter den Gruppen Aziridinyl, Azetidinyl, Pyrrolidinyl, Piperidinyl, Hexahydroazepinyl, Heptamethylenimin, Octamethylenimin, Morpholin und 4-Niederalkylpiperazidinyl, und die heterocyklischen Gruppen an ihren Kohlenstoffatomen durch 1 bis 3 Niederalkyl-, Hydroxy- oder Alkoxygruppen substituiert sein können ;

die Gruppe $R_4$ ausgewählt ist unter Wasserstoff, eine Alkylgruppe, Alkenyl, Alkoxyalkyl, Cykloalkyl, Aryl, Alkylaryl, Arylalkyl, Alkylarylalkyl, Alkoxyarylalkyl und niederen Haloarylalkyl, sowie die Additionssalze physiologisch annehmbarer Säuren.

2. Zusammensetzung nach Anspruch 1, dadurch gekennzeichnet, daß die Pyrimidinderivate ausgewählt sind unter den Verbindungen der Formel (III), worin $R_4$ Wasserstoff bedeutet, und $R_3$ eine Gruppe :

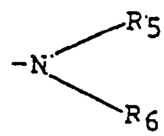

bedeutet, worin $R_5$ und $R_6$ einen Piperidinylring bilden oder eine Ethylgruppe sind, sowie ihre physiologisch annehmbaren Salze.

3. Zusammensetzung nach Anspruch 1 oder 2, dadurch gekennzeichnet, daß das Amphocarboxyglycinat der Formel (I) ausgewählt ist unter den Verbindungen der Formel (I), worin $R_1$ ein von Kopra abgeleitetes Alkylderivat bedeutet.

4. Zusammensetzung nach einem der Ansprüche 1 oder 2, dadurch gekennzeichnet, daß das Amphocarboxypropionat der Formel (II) ausgewählt ist unter den Verbindungen, worin bedeuten :

$R_2$ ein von Kopra abgeleitetes Alkylradikal
n = 2
X = $CH_2CH_2COONa$
Y = COONa

5. Zusammensetzung nach einem der Ansprüche 1, 2 und 3, dadurch gekennzeichnet, daß sie in einem physiologisch annehmbaren Milieu mindestens das 6-Amino-1,2-dihydro-1-hydroxy-2-imino-4-piperidino-pyrimidin enthält und ein amphoteres oberflächenaktives Mittel der Formel (I), worin $R_1$ ein von Kopra abgeleitetes Alkylradikal bedeutet.

6. Zusammensetzung nach einem der Ansprüche 1 bis 5, dadurch gekennzeichnet, daß die Pyrimidinderivate der Formel (III) in den erfindungsgemäßen Zusammensetzungen in Anteilen zwischen 0.05 und 6 Gew.%, bezogen auf das Gesamtgewicht der Zusammensetzung, vorhanden sind, und vorzugsweise von 0.1 bis 3 Gew.%.

7. Zusammensetzung nach einem der Ansprüche 1 bis 6, dadurch gekennzeichnet, daß das amphotere oberflächenaktive Mittel der Formel (I) oder (II) in der Zusammensetzung in Anteilen zwischen 1 und 30 Gew.%, bezogen auf das Gesamtgewicht der Zusammensetzung, vorhanden ist, und vorzugsweise zwischen 3 und 20 Gew.%.

8. Zusammensetzung nach einem der Ansprüche 1 bis 7, dadurch gekennzeichnet, daß das physiologisch annehmbare Milieu ein wässeriges Milieu ist oder eine Mischung aus Wasser und einem kosmetisch oder pharmazeutisch annehmbaren Lösungsmittel, verdickt oder nicht verdickt.

9. Zusammensetzung nach einem der Ansprüche 1 bis 8, dadurch gekennzeichnet, daß die Lösungsmittel in Anteilen zwischen 0.5 und 10 Gew.%, bezogen auf das Gesamtgewicht der Zusammensetzung, verwendet werden.

10. Zusammensetzung nach einem der Ansprüche 1 bis 9, dadurch gekennzeichnet, daß sie Verdickungsmittel enthält, ausgewählt aus den Heterobiopolysacchariden, den Cellulosederivaten, den vernetzten oder nicht vernetzten Acrylpolymeren, die in Anteilen zwischen 0.1 und 5 Gew.%, bezogen auf das Gesamtgewicht der Zusammensetzung, vorliegen.

11. Zusammensetzung nach einem der Ansprüche 1 bis 10, dadurch gekennzeichnet, daß die Zusammensetzung außerdem mindestens ein nicht-ionisches oberflächenaktives Mittel enthält.

12. Zusammensetzung nach Anspruch 11, dadurch gekennzeichnet, daß das nicht ionische oberflächenaktive Mittel ausgewählt ist aus den Polyhydroxypropylethern, den Fettalkohol-Polyoxyethylen-Ethern, den oxyethylenierten Alkylphenolen, den Mischungen von Fettalkoholen mit polyoxyethylenierten Fettalkoholen.

13. Zusammensetzung nach Anspruch 12, dadurch gekennzeichnet, daß das nicht-ionische oberflächenaktive Mittel vom Typ Polyhydroxypropylether ausgewählt ist aus :

(i) den Verbindungen der Formel :

$$R_7O-(CH_2-\underset{CH_2OH}{\underset{|}{CH}}O)_m-H \qquad (IV)$$

worin $R_7$ ein Alkylradikal oder eine Mischung von Alkylradikalen mit 10 bis 14 Kohlenstoffatomen bedeutet, und M eine Ganzeoder Dezimalzahl von 2 bis 10 ist ;

(ii) den Verbindungen der Formel :

$$R_8-CONH-CH_2-CH_2-O-CH_2-CH_2-O(CH_2-CHOH-CH_2-O)_n-H \qquad (V)$$

worin $R_8$ ein Alkyl oder Alkenylradikal oder eine Mischung aus Alkylradikalen und/oder Alkenylradikalen mit 11 bis 17 Kohlenstoffatomen bedeutet, und n eine Ganze- oder Dezimalzahl von 1 bis 5 ist ;

(iii) den Verbindungen der Formel :

$$R_9-CHOH-CH_2-O(CH_2-CHOH-CH_2-O)_p-H \qquad (VI)$$

worin $R_9$ ein aliphatisches, cykloaliphatisches, arylaliphatisches Radikal mit vorzugsweise 7 bis 21 Kohlenstoffatomen und ihre Mischungen bedeutet ;

(iv) den Verbindungen, hergestellt durch Kondensation, unter saurer Katalyse, von 2 bis 10 und vorzugsweise 2.5 bis 6 Molen Glycidol pro Mol Alkohol oder alpha-Diol mit 10 bis 14 Kohlenstoffatomen ;

(v) den Verbindungen, hergestellt durch (Poly)Addition von Glycerinmonochlorhydrin an eine organische Poly(Hydroxyl)Verbindung in Gegenwart einer starken Base und unter Wasserabspaltung.

14. Zusammensetzung nach Anspruch 12, dadurch gekennzeichnet, daß das nicht ionische oberflächenaktive Mittel aus der Gruppe der Polyethylenglycolether ausgewählt ist unter den Kondensationsprodukten von Fettalkoholen mit 10 bis 22 Kohlenstoffatomen mit 1 bis bis 24 Molen Ethylenoxid.

15. Zusammensetzung nach Anspruch 12, dadurch gekennzeichnet, daß das oxyethylenierte Alkylphenol ein oxyethyleniertes $C_8$ bis $C_9$-Alkylphenol mit 1 bis 100 Mol Ethylenoxid ist.

16. Zusammensetzung nach Anspruch 12, dadurch gekennzeichnet, daß die Mischung der Fettalkohole und polyoxyethylenierten Fettalkohole eine Mischung von $C_{10}$ bis $C_{22}$-Fettalkoholen und oxyethylenierten $C_{10}$ bis $C_{22}$-Fettalkoholen mit 2 bis 50 Mol Ethylenoxid ist.

17. Zusammensetzung nach einem der Ansprüche 1 bis 16, dadurch gekennzeichnet, daß sie außerdem Konservierungsmittel, Komplexierungsmittel, Farbstoffe, Alkalisierungsmittel oder Ansäurungsmittel, Parfums, Polymere enthält.

18. Zusammensetzung nach einem der Ansprüche 1 bis 17, dadurch gekennzeichnet, daß der pH-Wert zwischen 4 und 19 liegt.

19. Verfahren zur kosmetischen Behandlung der Haare, dadurch gekennzeichnet, daß man eine gemäß einem der Ansprüche 1 bis 17 definierte Zusammensetzung auf die nassen Haare appliziert, einmassiert, einige Minuten in Kontakt läßt, mit Wasser spült und gegebenenfalls erneut diese Zusammensetzung appliziert und danach erneut spült.

20. Zusammensetzung nach einem der Ansprüche 1 bis 18 zur Anwendung bei der therapeutischen Behandlung von Alopezie.

21. Verwendung der Zusammensetzung nach einem der Ansprüche 1 bis 18 zur Herstellung eines Medikamentes zur therapeutischen Behandlung von Alopezie.

**Claims**

1. Washing and foaming composition for inducing and stimulating hair growth and/or retarding its loss, characterized in that it contains, in a physiologically acceptable medium, at least one amphoteric surfactant selected from amphocarboxyglycinates of formula :

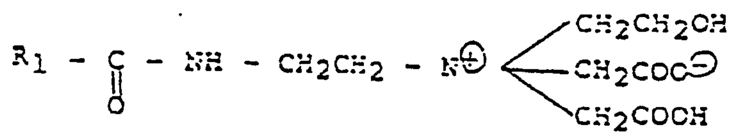

(I)

in which $R_1$ can denote an alkyl radical derived from coconut or a heptyl, nonyl or undecyl radical ; and amphocarboxypropionates corresponding to the formula :

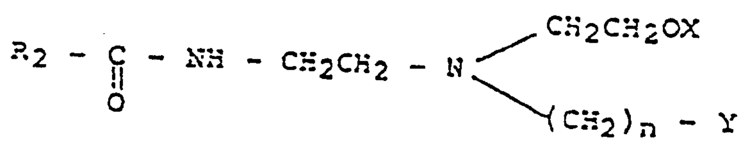

(II)

in which :

n = 1 or 2 ;

X denotes $CH_2CH_2COOH$ or hydrogen ;

Y denotes COOH or a

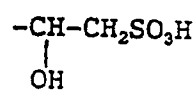

radical ; and

$R_2$ denotes an alkyl radical derived from coconut, a saturated $C_7$, $C_9$, $C_{11}$ or $C_{13}$ alkyl radical, a $C_{17}$ alkyl radical and its isoform, an unsaturated $C_{17}$ radical or an alkyl radical derived from linseed oil ; as well as the physiologically acceptable salts of these compounds ; and a pyrimidine derivative corresponding to the formula:

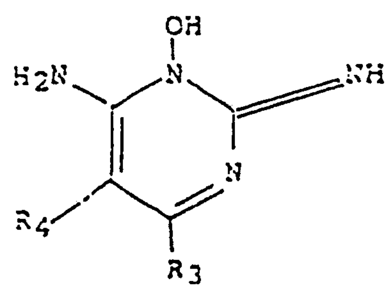

(III)

in which $R_3$ denotes a group

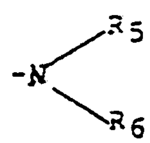

in which :

$R_5$ and $R_6$ are selected from hydrogen, an alkyl group preferably having 1 to 4 carbon atoms and a lower cycloalkyl, alkenyl or alkylaryl group ;

$R_5$ and $R_6$ can also form a heterocycle with the nitrogen atom to which they are attached, this heterocycle being selected from aziridinyl, azetidinyl, pyrrolidinyl, piperidyl, hexahydroazepinyl, heptamethylenimine,

octamethylenimine, morpholine and 4-(lower alkyl)piperazinyl, it being possible for the heterocyclic groups to be substituted on the carbon atoms with one to three lower alkyl, hydroxy or alkoxy groups ; and the group $R_4$, is selected from hydrogen and an alkyl, alkenyl, alkoxyalkyl, cycloalkyl, aryl, alkylaryl, arylalkyl, alkylarylalkyl, alkoxyarylalkyl and lower haloarylalkyl group ; as well as the physiologically acceptable addition salts with acids.

2. Composition according to Claim 1, characterized in that the pyrimidine derivatives are selected from the compounds of formula (III) in which $R_4$ , denotes hydrogen and $R_3$ denotes a group :

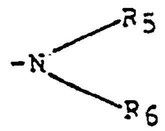

in which $R_5$ and $R_6$ form a piperidyl ring or alternatively an ethyl group, as well as their physiologically acceptable salts.

3. Composition according to Claim 1 or 2, characterized in that the amphocarboxyglycinate of formula (I) is selected from the compounds of formula (I) in which $R_1$ denotes an allyl radical derived from coconut.

4. Composition according to either of Claims 1 and 2, characterized in that the amphocarboxypropionate of formula (II) is selected from compounds in which :

$R_2$ denotes an alkyl radical derived from coconut

$n = 2$

$X = CH_2CH_2COONa$

$Y = COONa$

5. Composition according to any one of Claims 1, 2 and 3, characterized in that it contains, in a physioelogically acceptable medium, at least 6-amino-1,2-dihydro-1-hydroxy-2-imino-4-piperidinopyrimidine and an amphoteric surfactant of formula (I) in which $R_1$ denotes an alkyl radical derived from coconut.

6. Composition according to any one of Claims 1 to 5, characterized in that the pyrimidine derivatives of formula (III) are present in the compositions according to the invention in proportions of between 0.05 and 6% by weight relative to the total weight of the composition, and preferably from 0.1 to 3% by weight.

7. Composition according to any one of Claims 1 to 6, characterized in that the amphoteric surfactant of formula (I) or (II) is present in the composition in proportions of between 1 and 30% by weight relative to the total weight of the composition, and preferably between 3 and 20% by weight.

8. Composition according to any one of Claims 1 to 7, characterized in that the physiologically acceptable medium is an aqueous medium or a mixture of water and a solvent, cosmetically or pharmaceutically acceptable, thickened or otherwise.

9. Composition according to any one of Claims 1 to 8, characterized in that the solvents are used in proportions of between 0.5 and 10% by weight relative to the total weight of the composition.

10. Composition according to any one of Claims 1 to 9, characterized in that it contains thickeners selected from heterobiopolysaccharides, cellulose derivatives and acrylic polymers, crosslinked or otherwise, present in proportions of between 0.1 and 5% by weight relative to the total weight of the composition.

11. Composition according to any one of Claims 1 to 10, characterized in that the composition contains, in addition, at least one nonionic surfactant.

12. Composition according to Claim 11, characterized in that the nonionic surfactant is selected from polyhydroxypropyl ethers, ethers of polyoxyethylenated fatty alcohols, oxyethylenated alkylphenols and mixtures of fatty alcohols and polyoxyethylenated fatty alcohols.

13. Composition according to Claim 12, characterized in that the nonionic surfactant of the polyhydroxypropyl ether type is selected from :

(i) the compounds of formula :

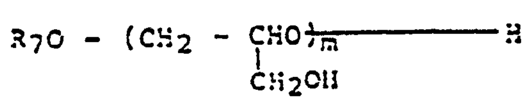

(IV)

in which $R_7$ denotes an alkyl radical or mixture of alkyl radicals containing 10 to 14 carbon atoms and m is an integer or decimal number from 2 to 10 ;

(ii) the compounds of formula :

$$R_8-CONH-CH_2-CH_2-O-CH_2-CH_2-O(CH_2-CHOH-CH_2-O)_n-H \quad (V)$$

in which $R_8$ denotes an alkyl or alkenyl radical or mixture of alkyl and/or alkenyl radicals having from 11 to 17 carbon atoms and n denotes an integer or decimal number from 1 to 5 ;

(iii) the compounds of formula :

$$R_9-CHOH-CH_2-O(CH_2-CHOH-CH_2-O)_p-H \quad (VI)$$

in which $R_9$ denotes an aliphatic, cycloaliphatic or arylaliphatic radical preferably having 7 to 21 carbon atoms, and mixtures thereof ;

(iv) the compounds prepared by the acid-catalysed condensation of 2 to 10, and preferably 2.5 to 6, moles of glycidol per mole of alcohol or alpha-diol, containing 10 to 14 carbon atoms ; and

(v) the compounds prepared by the (poly)addition of glycerol monochlorohydrin to a poly(hydroxylated) compound in the presence of a strong base and elimination of water.

14. Composition according to Claim 12, characterized in that the nonionic surfactant of the polyethylene glycol ether family is selected from the condensation products of fatty alcohols having 10 to 22 carbon atoms with 1 to 24 moles of ethylene oxide.

15. Composition according to Claim 12, characterized in that the oxyethylenated alkylphenol is a ($C_8$-$C_9$ alkyl)phenol oxyethylenated with 1 to 100 moles of ethylene oxide.

16. Composition according to Claim 12, characterized in that the mixture of fatty alcohols and polyoxyethylenated fatty alcohols is a mixture of $C_{10}$-$C_{22}$ fatty alcohols and $C_{10}$-$C_{22}$ fatty alcohols oxyethylenated with 2 to 50 moles of ethylene oxide.

17. Composition according to any of Claims 1 to 16, characterized in that it also contains preservatives, complexing agents, colourings, alkalinizing or acidifying agents, fragrances, polymers.

18. Composition according to any one of Claims 1 to 17, characterized in that the pH is between 4 and 9.

19. Process for cosmetic treatment of the hair, characterized in that the composition defined in any one of Claims 1 to 17 is applied on wet hair, in that massaging is performed, in that the composition is left in contact for a few minutes, in that the hair is rinsed with water and in that this composition is optionally applied once more, followed by a further rinse.

20. Composition according to any one of Claims 1 to 18, for its application in the therapeutic treatment of alopecia.

21. Use of the composition according to any one of Claims 1 to 18 for the preparation of a medicinal product intended for the therapeutic treatment of alopecia.